# EUROPÄISCHE PATENTSCHRIFT

(11) **EP 1 852 822 B1**
(45) Veröffentlichungstag und Bekanntmachung des Hinweises auf die Patenterteilung: **12.08.2020**
(21) Anmeldenummer: 07106614.6
(22) Anmeldetag: 20.04.2007
(51) Int. Cl.: G06T 1/00, G06T 7/00, A61B 6/00

(54) **Generierung eines dreidimensionalen medizinischen Bildes mit unabhängiger Positionierung von Emissionsquelle und Detektor**
Generation of a three-dimensional medical image with independent positioning of emission source and detector
Génération d'image médicale tridimensionnelle avec positionnement indépendant de source d'émission et de détecteur

(30) Priorität: 05.05.2006 DE 102006021051; 05.05.2006 US 798264 P
(43) Veröffentlichungstag der Anmeldung: 07.11.2007
(73) Patentinhaber: Siemens Healthcare GmbH, 91052 Erlangen (DE)
(72) Erfinder: Herrmann, Klaus, 90403 Nürnberg (DE); Rietzel, Eike, 64289 Darmstadt (DE)

(56) Entgegenhaltungen:
- WO-A1-00/04830
- US-A- 5 475 730
- US-A- 5 835 558
- HEMLER P F ET AL: "Improved 3D reconstructions for generalized tomosynthesis", PROCEEDINGS OF THE SPIE - THE INTERNATIONAL SOCIETY FOR OPTICAL ENGINEERING SPIE-INT. SOC. OPT. ENG. USA, Bd. 5030, 2003, Seiten 361-370, XP002743352, ISSN: 0277-786X
- S Siltanen, V Kolehmainen et al: "Statistical inversion for medical x-ray tomography with few radiographs: I. General theory", INSTITUTE OF PHYSICS PUBLISHING , 2003, XP002743353, Gefunden im Internet: URL:http://iopscience.iop.org/0031-9155/48 /10/315/pdf/0031-9155_48_10_315.pdf [gefunden am 2015-08-14]
- DOBBINS J T ET AL: "Digital x-ray tomosynthesis: current state of the art and clinical potential", PHYSICS IN MEDICINE AND BIOLOGY, INSTITUTE OF PHYSICS PUBLISHING, BRISTOL GB, Bd. 48, Nr. 19, 7. Oktober 2003 (2003-10-07), Seiten R65-R106, XP002277335, ISSN: 0031-9155, DOI: 10.1088/0031-9155/48/19/R01
- Avinash C. Kak, Malcolm Slaney: "Principles Computerized Tomographic Imaging", , 1999, XP002753293, New Yorck, USA Gefunden im Internet: URL:http://www.slaney.org/pct/ [gefunden am 2016-01-18]
- LA RIVI GBP ERE P J ET AL: "Few-View Tomography Using Roughness-Penalized Nonparametric Regression and Periodic Spline Interpolation", IEEE TRANSACTIONS ON NUCLEAR SCIENCE, IEEE SERVICE CENTER, NEW YORK, NY, US, Bd. 46, Nr. 4, 1. August 1999 (1999-08-01) , Seiten 1121-1128, XP011088478, ISSN: 0018-9499, DOI: 10.1109/23.790845

## Beschreibung

Die Erfindung bezieht sich auf ein Verfahren zur Generierung eines medizinischen Bildes mit mindestens einer Emissionsquelle und mindestens einem Detektor eines medizinischen Bildaufnahmesystems, wobei die Strahlung der Emissionsquelle das Objekt durchdringt und die von einem Objekt Abschwächbahre Strahlung vom Detektor detektiert und die vom Detektor detektierte Strahlung zur Generierung des medizinischen Bildes dient. Die Erfindung bezieht sich ebenfalls auf ein medizinisches Bildaufnahmesystem zur Erzeugung von Bilddaten als Grundlage zur Generierung eines medizinischen Bildes.

Aus dem Stand der Technik sind Bildgebungssysteme, wie z.B. Computertomographen, auf der Basis von Röntgengeräten bekannt. So offenbart die DE 197 46 093 A1 ein Röntgensystem mit einer Röntgenstrahlungsquelle und einem Röntgenstrahlungsempfangseinrichtung, wobei das Röntgensystem zur Aufnahme von zweidimensionalen Projektionen und anschließender dreidimensionaler Bildrekonstruktion dient, wobei die zur Bildrekonstruktion erforderlichen Projektionswinkel durch Sende- und Empfangseinrichtungen für Schallwellen oder elektromagnetische Wellen ermittelt werden.

Die Emissionsquellen herkömmlicher dreidimensionaler Bildgebungssysteme sind zumeist Röntgenquellen, die ebenso wie der korrespondierend ausgerichtete Detektor, bezüglich eines Isozentrums angeordnet sind. Die Rotationsachsen der Emissionsquelle und des zumeist um 180° versetzt mitlaufenden Detektors sind bisher so angeordnet, dass diese durch die gedachte Linie des Strahlenverlaufes von der Emissionsquelle bis zur Mitte des Detektors verläuft. Der Überlappungsbereich der möglichen Strahlenverläufe bei diesen herkömmlichen Bildgebungssystemen bildet einen Volumenbereich als so genanntes Rekonstruktionsvolumen, der vom Bildgebungssystem erfasst und Grundlage für eine anschließende 3D-Bildverarbeitung in Form eines 3D-Datensatzes ist. Auf der Basis einer definierten Anzahl von Durchleuchtungsbildern mit vorgegebenen Winkelabständen während einer orbitalen Rotation um 190° erzeugt beispielsweise das Bildgebungssystem "SIREMOBIL Iso-C3D" ein würfelförmiges dreidimensionales Rekonstruktionsvolumen mit einer Kantelänge von bis zu 12cm ("Klinische Studie zur registrierungsfreien 3D-Navigation mit dem mobilen C-Bogen SIREMOBIL Iso-C3D", P. A. Grützner, A. Hebecker, H. Waelti, B. Vock, L.-P. Nolte, A. Wentzensen, electromedica 71, Heft 1, Seite 58-67, 2003).

Aufgrund dieser Einschränkung des dreidimensionalen Rekonstruktionsvolumens ist es bisher nur möglich, entweder das Bildgebungssystem so zu vergrößern, dass ein vergrößertes Volumen das zu untersuchende Objekt durchleuchtet und damit ein entsprechend großer Volumenbilddatensatz erzeugt wird. Alternativ ist es, insbesondere bei mobilen Bildausnahmensystemen wie Röntgen C-Bögen, möglich, das Bildaufnahmesystem auf die neue Untersuchungsregion auszurichten. Diese Neuausrichtung des mobilen Bildgebungssystems auf eine weitere Untersuchungsregion des Objektes erfordert jedoch einige Erfahrung vom Bediener und ist insbesondere während einer Operation zur Überwachung des Operationsfortschrittes unpraktikabel.

Aus Hemler et al., "Improved 3D reconstructions for generalized tomosynthesis", Medical Imaging 2003: Physics of Medical Imaging, M. J. Yaffe, L. E. Antonuk (Editors), Proceedings of SPIE, Vol. 5030 (2003) is ein Verfahren für die "Tuned-Aperture Computed Tomography" bekannt, bei der die Emissionsquelle frei zu einem Detektor positioniert werden kann. Die bei der Bilderfassung unbekannte Aufnahmegeometrie wird nachträglich anhand der Lage von Markern bestimmt, die in allen erfassten Projektionen gezeigt sein muss.

Aus WO 00/04830 A1 ist ein Verfahren bekannt, bei dem ein Volumendatensatz eines Objektes anhand von Sätzen von Projektionsaufnahmen mittels Tomosynthese rekonstruiert wird. Dabei können insbesondere erste und zweite Volumendatensätze kombiniert oder fusioniert werden.

Der Erfindung liegt die Aufgabe zugrunde, eine Möglichkeit zur Generierung von medizinischen Bildern bereitzustellen, die auf der Grundlage von 2D-Röntgensystemen insbesondere in der Onkologie und Angiographie, sowie auf der Grundlage von mobilen Bildaufnahmesystemen in der Chirurgie eine vollständige Abbildung des Untersuchungsobjektes in einem Volumenbilddatensatz erlaubt, ohne dass die baulichen Abmessungen der insbesondere mobilen Bildaufnahmesysteme vergrößert werden müssen.

Diese Aufgabe wird erfindungsgemäß durch ein Verfahren zur Generierung eines medizinischen Bildes mit den Merkmalen des Anspruchs 1 und durch ein medizinisches Bildaufnahmesystem mit den Merkmalen des Anspruchs 4 gelöst.

Das Verfahren zur Generierung des medizinischen Bildes erfolgt mittels eines medizinischen Bildaufnahmesystems, das eine unabhängige Positionierung einer Emissionsquelle und eines Detektors des medizinischen Bildaufnahmesystems bei jeder Objektbestrahlung relativ zueinander vorsieht, wobei zwischen der Emissionsquelle und dem Detektor zumindest ein Teil des zu untersuchenden Objektes angeordnet ist und zumindest ein Teil der Strahlung der Emissionsquelle vom Detektor detektiert wird.

Im Sinne der Erfindung ist ein Objekt ein durch das medizinische Bildaufnahmesystem untersuchbarer Volumenkörper, insbesondere der Körper eines menschlichen oder tierischen Patienten.

Nach der Detektion der Objektbestrahlung als Ermittlung der zum Teil abgeschwächten Strahlung während der Durchleuchtung des Objektes zu einem bestimmten Zeitpunkt wird gleichzeitig die jeweilige Position der Emissionsquelle und des Detektors bei jeder Objektbestrahlung ermittelt. Die Kenntnis der genauen Position der Emissionsquelle und des Detektors bei jeder Objektbestrahlung relativ zueinander und im Bezug auf das Objekt ist für die Bildung des Volumenbilddatensatzes, z.B. als CT-Bilddatensatz, und damit für die Bildrekonstruktion unerlässlich.

Die Gesamtheit der Objektbestrahlungen definiert mindestens zwei Überlappungsbereiche als dreidimensionale Rekonstruktionsvolumen, der der Untersuchungsregion des Objektes entspricht. Der Volumenbilddatensatz wird auf der Grundlage der dreidimensionalen Rekonstruktionsvolumen unter Berücksichtigung der zugehörigen Positionen der Emissionsquelle und des Detektors bei jeder Objektbestrahlung gebildet. Abschließend wird das medizinische Bild bezüglich mindestens einer vorgegebener Schnittebene aus dem Volumenbilddatensatz mittels bekannter Bildverarbeitungsverfahren extrahiert.

Ein wesentlicher Vorteil des erfindungsgemäßen Verfahrens besteht darin, dass durch die unabhängige Positionierung der Emissionsquelle und des Detektors ein nahezu beliebiges Volumen als Überlappungsbereich der Objektbestrahlungen gewählt werden kann und nur durch die baulichen Abmessung des Bildaufnahmesystems zwischen Emissionsquelle und Detektor beschränkt ist. Stationäre Bildaufnahmesysteme in der Onkologie und Angiographie sowie mobile Bildaufnahmesysteme in der Chirurgie besitzen herkömmlicherweise nur einen Drehpunkt des Bildaufnahmesystems aufgrund der isozentrischen Anordnung der Emissionsquelle und des Detektors und erzeugen damit nur ein eng begrenztes Rekonstruktionsvolumen. Ein Beispiel für eine mobile Röntgenvorrichtung, die es theoretisch ermöglicht, den Drehpunkt frei zu wählen, findet sich z.B. in der amerikanischen Patentspezifikation US 5475730. Durch das erfindungsgemäße Verfahren werden das Rekonstruktionsvolumen und damit der Volumenbilddatensatz deutlich vergrößert. Mehrmalige Ausrichtungen eines mobilen oder stationär betriebenen Bildaufnahmesystems entfallen beim erfindungsgemäßen Verfahren und vergrößern- bei unveränderten baulichen Abmessungen des Bildaufnahmesystems - gleichzeitig die Größe der untersuchbaren Region des Objektes.

In einer vorteilhaften Ausgestaltung des Verfahrens werden Bildverzerrungen der im Detektor detektierten Objektbestrahlung bei der Erzeugung der dreidimensionalen Rekonstruktionsvolumen berücksichtigt. Für den Fall einer Schrägstellung des Detektors im Bezug auf die Richtung des jeweiligen Zentralstrahls der Emissionsquelle entstehen geometrische Bildverzerrungen, die zu einem fehlerhaften Bildergebnis führen. Da das Rekonstruktionsvolumen Grundlage für die Bildung des Volumenbilddatensatzes ist, beeinflussen die Bildverzerrungen die Qualität des Volumenbilddatensatzes und damit auch die Qualität der hieraus generierten medizinischen Bilder. Die Ermittlung dieser Bildverzerrungen anhand der Position der Emissionsquelle relativ zur Detektorfläche wird beim erfindungsgemäßen Verfahren bei der Erzeugung des mit dem Rekonstruktionsvolumen verbundenen Bilddatensatzes berücksichtigt. Durch diese Maßnahmen hat auch eine nicht optimale relative Positionierung der Emissionsquelle relativ zum Detektor keinen Einfluss auf die Qualität des Bildergebnisses.

Erfindungsgemäß ist vorgesehen, dass die Emissionsquelle und der Detektor dergestalt zueinander bei jeder Objektbestrahlungen angeordnet werden, dass die Objektbestrahlungen mindestens zwei Überlappungsbereiche und damit mindestens zwei dreidimensionale Rekonstruktionsvolumen des Objektes bilden. Vorteilhafterweise werden auf der Basis der dreidimensionalen Rekonstruktionsvolumen jeweils Volumenbilddatensätze erzeugt, wobei die so gebildeten Volumenbilddatensätze mittels graphischer Bilddatenverarbeitungsverfahren zu einem gesamten Volumenbilddatensatz kombiniert werden. Die Kombination der Bilddatensätze kann auf der Ebene der einzeln gebildeten Volumenbilddatensätze erfolgen.

Erfindungsgemäß werden die einzelnen Volumenbilddatensätze mit einem graphischen Bilddatenverarbeitungsverfahren, wie z.B. ein Bild-Stitching-Verfahren, kombiniert. Dieses insbesondere in der digitalen Bildverarbeitung genutzte Verfahren dient dazu, Bilddaten von einzelnen Bildaufnahmen eines Objektes so zu kombinieren (engl. stitch = nähen, zusammenheften), dass die einzelnen Bildaufnahmen zu einer Gesamtaufnahme des Objektes verbindbar sind. Beispielsweise werden somit aus Einzelbildern mit unterschiedlichen Aufnahmewinkeln eines Objektes so genannte Panoramabilder, wie z.B. von Gebäuden, erstellt. Durch die Verwendung dieser graphischen Bilddatenverarbeitungsverfahren können die einzelnen Volumenbilddatensätze zu entsprechenden Gesamtbilddatensätzen verbunden werden. Hierdurch ergibt sich die Möglichkeit, das gesamte Objekt durch einen Volumenbilddatensatz abzubilden und die Generierung von Schnittebenenbildern bezogen auf das gesamte Volumen des Objektes als medizinische Bilder auszuwerten.

Die Erfindung bezieht sich außerdem auf ein medizinisches Bildaufnahmesystem mit mindestens einer Emissionsquelle zur Erzeugung einer Strahlung und einem Detektor zur Detektion der Strahlung, wobei die vom Detektor detektierte Strahlung zur Generierung eines medizinischen Bildes verwendbar ist.

Erfindungsgemäß ist vorgesehen, dass die relative Position und Ausrichtung der Emissionsquelle und des Detektors bezogen auf einen frei wählbaren Drehpunkt durch mindestens ein Positionierungselement erfolgt, wobei eine Steuerung gewährleistet, dass die Emissionsquelle und der Detektor so positionierbar sind, dass zumindest ein Teil der Strahlung der Emissionsquelle auf den Detektor trifft. Vorteilhafterweise kann das Positionierungselement die Emissionsquelle und/oder den Detektor frei positionieren, wie dies z.B. mit einem mehrachsigen Roboter möglich ist.

In einer vorteilhaften Ausgestaltung des medizinischen Bildaufnahmesystems sind die Emissionsquelle und der Detektor auf einem C-Bogen angeordnet und der C-Bogen ist mittels des Positionierungselementes vertikal und/oder waagerecht verschiebbar.

Ebenfalls sind vorteilhafterweise zumindest zwei Positionierungselemente durch mindestens ein drehbar gelagertes Kopplungselement miteinander verbunden und gewährleisten hierdurch eine freie Positionierung der Emissionsquelle und des Detektors bezogen auf einen frei wählbaren Drehpunkt.

Weitere vorteilhafte Maßnahmen sind in den übrigen Unteransprüchen beschrieben; die Erfindung wird anhand von Ausführungsbeispielen und den nachfolgenden Figuren näher beschrieben. Es zeigen:
- FIG 1: eine schematische Schnittdarstellung des dreidimensionalen Rekonstruktionsvolumens aufgrund der isozentrischen Anordnung der Emissionsquelle und des Detektors gemäß dem bekannten Stand der Technik;
- FIG 2a, 2b: eine schematische Schnittdarstellung von zwei dreidimensionalen Rekonstruktionsvolumen aufgrund der Anwendung des erfindungsgemäßen Verfahrens;
- FIG 3: eine schematische Schnittdarstellung eines dreidimensionalen Rekonstruktionsvolumens von der Größe des zu untersuchenden Objektes aufgrund der freien Positionierung der Emissionsquelle und des Detektors gemäß dem erfindungsgemäßen Verfahren;
- FIG 4: eine schematische Darstellung des erfindungsgemäßen Verfahrens;
- FIG 5: eine Darstellung der Bildung der Projektionsmatrix gemäß dem erfindungsgemäßen Verfahren auf der Basis der Auswertung von drei Objektbestrahlungen;
- FIG 6: eine schematische Darstellung eines erfindungsgemäßen mobilen Bildaufnahmesystems mit einem vertikal und waagerecht verstellbaren, isozentrischen C-Bogen;
- FIG 7: eine schematische Darstellung eines erfindungsgemäßen Bildaufnahmesystems mit drehbar gelagerten Positionselementen.

In den Figuren sind sinngemäß gleiche Merkmale mit den gleichen Bezugszeichen versehen.

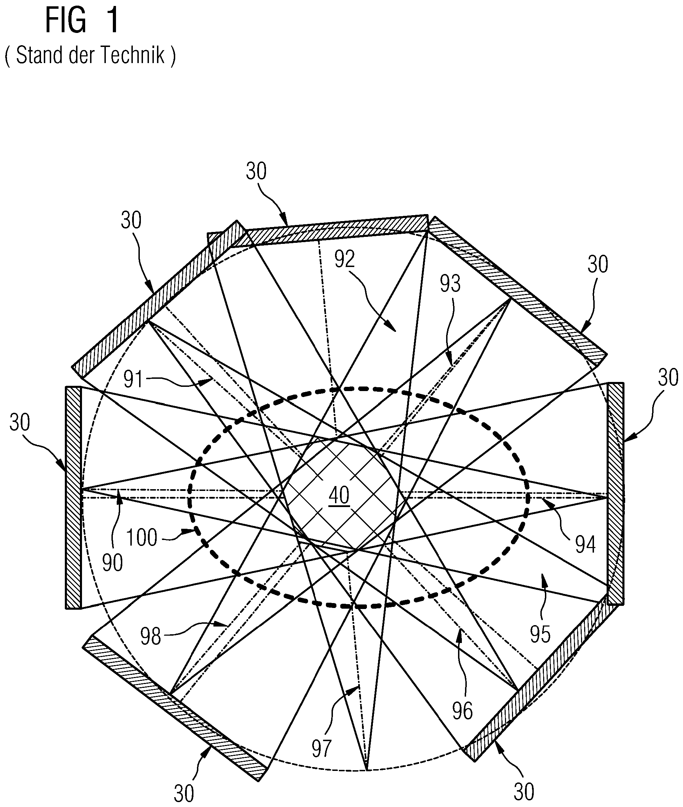

In der FIG 1 ist der bisherige Stand der Technik dargestellt. Herkömmlicherweise ist die Emissionsquelle 20 (nicht dargestellt) und der Detektor 30 in Form einer Fläche um ein Isozentrum drehbar angeordnet. Dies bedingt eine Bewegung der Emissionsquelle 20 und des Detektors 30 auf einer Kreisbahn, die als gestrichelter Kreisumfang in der Zeichnung FIG 1 eingezeichnet ist. Die Emissionsquelle 20 erzeugt eine Strahlung und emittiert diese bezüglich unterschiedlicher Winkel, die als Objektbestrahlung 90 bis 96 von dem korrespondierend mitrotierenden Detektor 30 jeweils detektiert werden. Der Strahlverlauf ist kegelförmig dargestellt, wobei im spitzen Winkel des Strahlverlaufes der Objektbestrahlungen 90 bis 98 die nicht dargestellte Emissionsquelle 20 die Strahlung erzeugt. Der Überlappungsbereich dieser Objektbestrahlungen 90 bis 96 in der Nähe des Isozentrums bildet das dreidimensionale Rekonstruktionsvolumen 40, wobei zur Bildung des dreidimensionalen Rekonstruktionsvolumens 40 auch Strahlenverläufe außerhalb der Zeichnungsebene das Objekt 100 durchstrahlen müssen.

Zur besseren Darstellung werden in den Zeichnungen nur einige ausgesuchte Objektbestrahlung 90 bis 99 in der Zeichenebene dargestellt. Zur Generierung eines Volumenbilddatensatzes werden Bilddaten von einer Vielzahl von Strahlenverläufen aus unterschiedlichen Aufnahmepositionen und -winkeln benötigt, so dass vorliegend die dargestellten Objektbestrahlungen 90 bis 99 lediglich als ausgesuchte repräsentative Strahlenverläufen zur besseren Beschreibung der Erfindungsidee anzusehen sind.

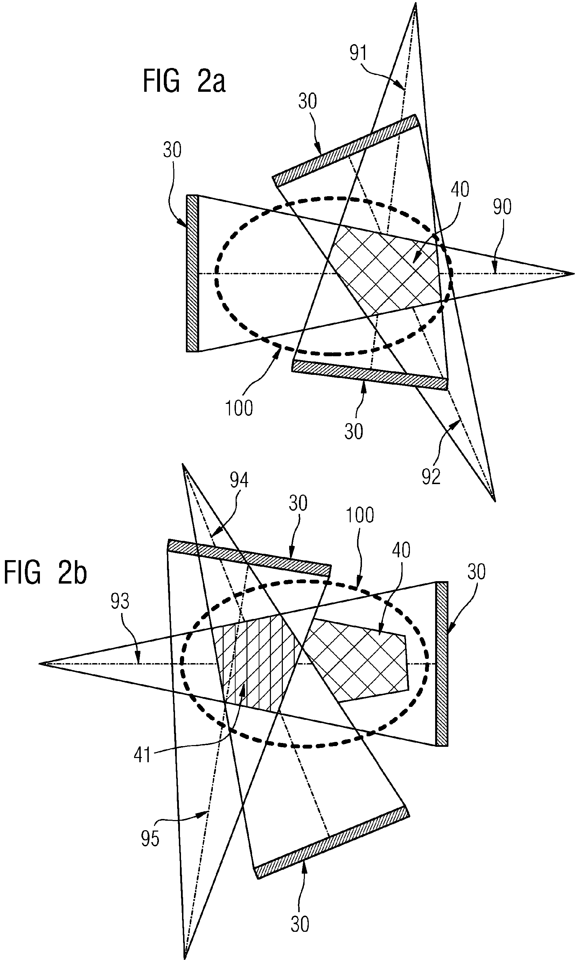

In den FIG 2a und FIG 2b ist die Erzeugung von zwei dreidimensionalen Rekonstruktionsvolumen 40, 41 gemäß dem erfindungsgemäßen Verfahren visualisiert. Das Objekt 100 wird durch eine freie Positionierung der Emissionsquelle 20 (nicht dargestellt) und des Detektors 30 von der Objektbestrahlung 90 bis 99 durchleuchtet. Dabei werden in einem ersten Schritt die Emissionsquelle 20 und der Detektor 30 so um das Objekt positioniert, dass ein erstes dreidimensionales Rekonstruktionsvolumen 40 durch die entsprechenden Objektbestrahlungen 90 bis 92 erzeugt wird (FIG 2a). In einem zweiten Schritt gemäß dem erfindungsgemäßen Verfahren werden die Emissionsquelle 20 und der Detektor 30 bezüglich einer zweiten Untersuchungsregion ausgerichtet und bilden somit ein zweites dreidimensionales Rekonstruktionsvolumen 41 (FIG 2b), das vom ersten dreidimensionalen Rekonstruktionsvolumen 40 innerhalb des Objektes 100 abweicht. Die dreidimensionalen Rekonstruktionsvolumen 40,41 dienen als Grundlage zur Bildung von zwei Volumenbilddatensätzen 50,51 (nicht dargestellt), die dann anschließend zu einem gesamten Volumenbilddatensatz 50 kombinierbar sind.

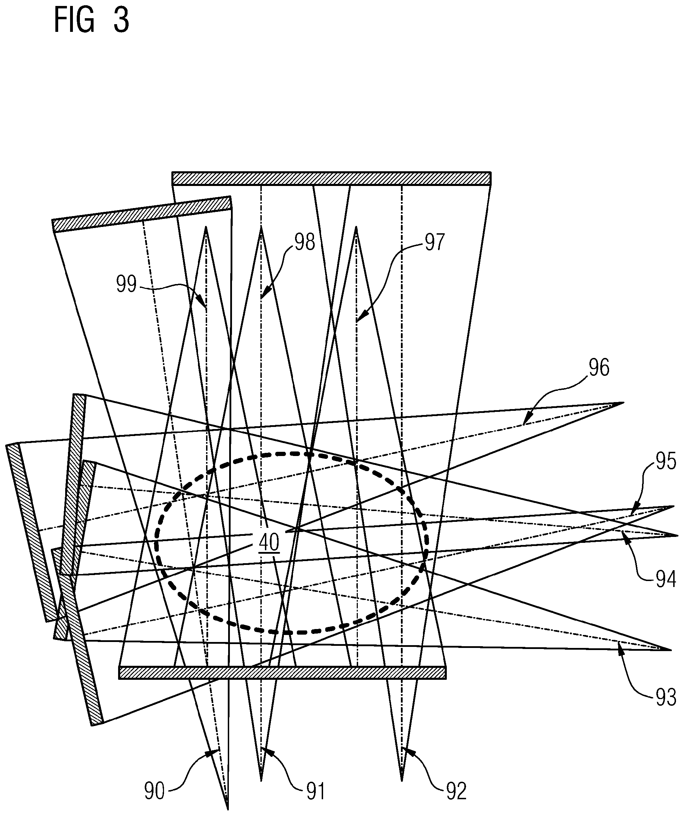

Die FIG 3 zeigt eine schematische Schnittdarstellung eines dreidimensionalen Rekonstruktionsvolumens 40 von der Größe des zu untersuchenden Objektes 100 aufgrund der freien Positionierung der Emissionsquelle 20 (nicht dargestellt) und des Detektors 30 gemäß einem nicht-erfindungsgemäßen Verfahren. Durch die vollständige Abdeckung des Volumens des Objektes 100 mit Objektbestrahlungen 90 bis 99 ergibt sichnicht nur ein dreidimensionales Rekonstruktionsvolumen 40 in der unmittelbaren Nähe eines Isozentrums, sondern es ist ein dreidimensionales Rekonstruktionsvolumen 40 von der Größe des Objektes 100 erzeugbar. Alleine der Abstand zwischen der Emissionsquelle 20 und dem Detektor 30 begrenzt die mögliche Größe des dreidimensionalen Rekonstruktionsvolumens 40 entsprechend dem erfindungsgemäßen Verfahren.

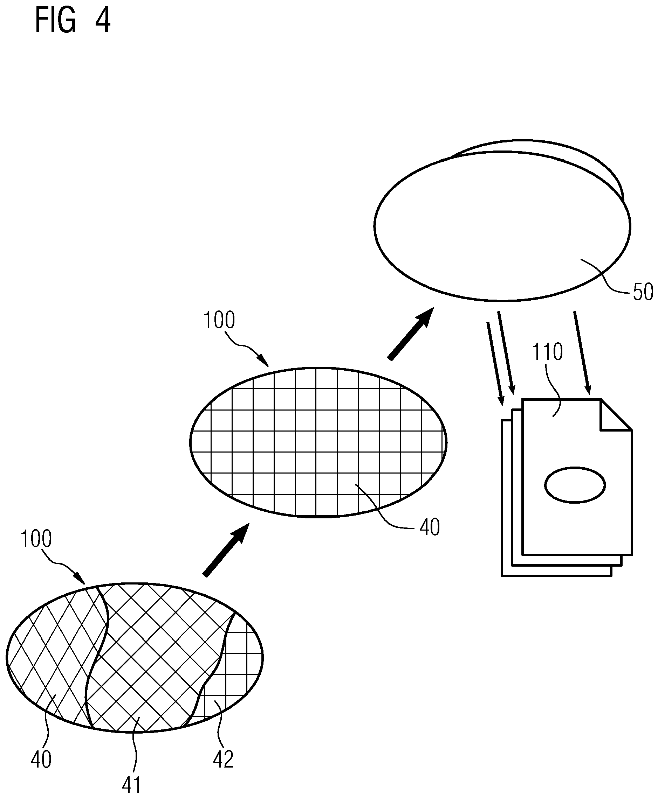

In dem Ablaufschema entsprechend der FIG 4 sind die wesentlichen Schritte des erfindungsgemäßen Verfahrens für eine Erzeugung von mehreren dreidimensionalen Rekonstruktionsvolumen 40 bis 42 mit anschließender Bildverarbeitung dargestellt. Durch eine entsprechende Positionierung der Emissionsquelle 20 (nicht dargestellt) und des Detektors 30 (nicht dargestellt) wird das zu untersuchende Objekt 100 mit Objektbestrahlungen 90 bis 99 (nicht dargestellt) vollständig abgedeckt, wobei im dargestellten Beispiel die Überlappungsbereiche der Objektbestrahlungen 90 bis 99 drei dreidimensionale Rekonstruktionsvolumen 40,41,42 erzeugen. Mittels eines Bilddatenverarbeitungsverfahrens, wie z.B. dem Stichting-Verfahren, werden die Bilddaten der Rekonstruktionsvolumen 40,41,42 zu einem Rekonstruktionsvolumen 40 - bezogen auf das gesamte Objekt 100 - zusammengeführt. Diese Bilddaten des Rekonstruktionsvolumens 40 dienen dann als Grundlage zur Erzeugung des Volumenbilddatensatzes 50, aus dem die medizinischen Bilder 110 als Ebenenschnittbilder aus dem Volumenbilddatensatz 50 extrahiert werden können. Bei der Bildverarbeitung der Bilddaten der dreidimensionalen Rekonstruktionsvolumen 40,41,42 werden ebenfalls geometrische Verzerrung bei der Aufnahme der Objektbestrahlungen 90 bis 99 durch den Detektor 30 bei der Bilddatenverarbeitung berücksichtigt und kompensiert.

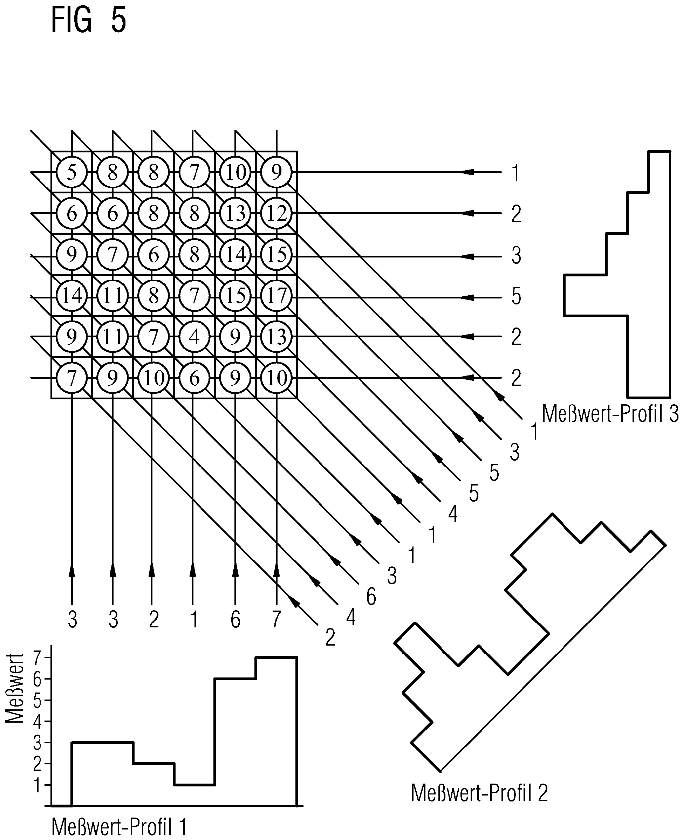

Die FIG 5 zeigt eine Darstellung der Bildung der Projektionsmatrix gemäß dem erfindungsgemäßen Verfahren auf der Basis der Auswertung von drei Objektbestrahlungen 90 bis 92 (nicht dargestellt). Die im Detektor 30 (nicht dargestellt) detektierten Messprofile von drei Objektbestrahlungen 90 bis 92 werden unter Berücksichtigung des jeweiligen Detektionswinkels in die Projektionsmatrix rückprojeziert. Die einzelnen Messprofile zeigen das Maß der Abschwächung der von der Emissionsquelle 30 (nicht dargestellt) ausgesandten und durch die spezifische Struktur des Objektes 100 (nicht dargestellt) abgeschwächten Strahlung. Idealerweise bildet die Projektionsmatrix das gesamte Volumen des Objektes 100 ab. Auch mögliche Verzerrungen, die zu einer Beeinträchtigung der Messprofile führen können, werden bei der Bildverarbeitung in der Projektionsmatrix, z.B. durch ortsabhängige Filteralgorithmen, mitberücksichtigt.

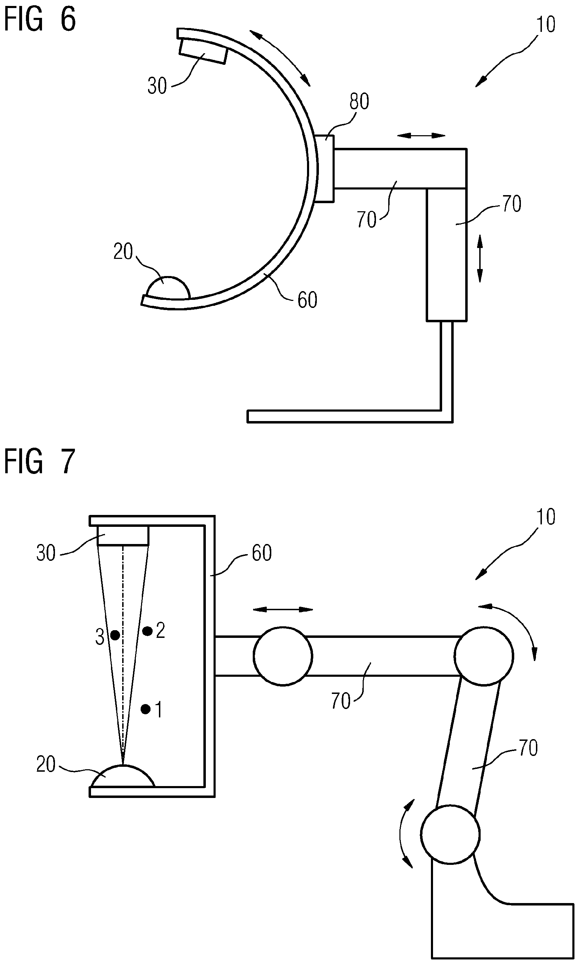

Die FIG 6 zeigt eine schematische Darstellung eines erfindungsgemäßen mobilen Bildaufnahmesystems 10. Ein herkömmlicher C-Bogen 60 mit fest verbundenerer Emissionsquelle 20 und fest verbundenem Detektor 30 ist mittels eines C-Bogenpositionierungselements 80 bezüglich eines nicht eingezeichneten Isozentrums um die orbitale Achse des C-Bogens 60 drehbar. Der C-Bogen 60 ist mit zwei Positionierungselementen verbunden und über die Positionierungselemente 70 vertikal und/oder waagerecht verschiebbar. Durch die Möglichkeit der vertikalen und/oder waagerechten Verschiebung des isozentrischen C-Bogens 60 durch die Positionierungselemente 70 ist eine freie Wahl des Drehpunktes des isozentrischen C-Bogens 60 innerhalb der Bildebene der FIG 6 möglich. Durch zusätzliche Positionierungselemente 70 mit normaler Ausrichtung zur Bildebene der FIG 6 (nicht eingezeichnet) ist darüber gewährleistet, dass Bildaufnahmen bezüglich eines Drehpunkts außerhalb der Bildebene durch das erfindungsgemäße medizinische Bildaufnahmesystem 10 aufgenommen werden können. Hierdurch vergrößert sich das von den überlappenden Strahlenverläufen gebildete dreidimensionale Rekonstruktionsvolumen 40 (nicht dargestellt).

Die FIG 7 zeigt ein erfindungsgemäßes Bildaufnahmesystem 10 mit einer auf einer U-förmigen Halterung angeordneten Emissionsquelle 20 und einem - bezogen die Mittelachse des Strahlverlaufes der Emissionsquelle 20 - korrespondierend angeordneten Detektor 30. Die U-förmige Halterung ist mit einem ersten Positionierungselement 70 verbunden. Das erste Positionierungselement 70 ist über ein drehbar gelagertes Kopplungselement mit einem zweiten Positionierungselement 70 verbunden, so dass die Positionierungselemente 70 relativ zueinander unterschiedliche Winkelstellungen einnehmen können. Darüber hinaus ist nicht nur die relative Lage der Positionierungselemente 70, sondern auch die Länge der jeweiligen Positionierungselemente 70 variabel und frei einstellbar. Das zweite Positionierungselement 70 ist weiterhin über ein zweites drehbar gelagertes Kopplungselement mit einem dritten Positionierungselement 70 drehbar verbunden. Eine Steuerung kann beispielsweise über in den Kopplungselementen angeordnete Schrittmotoren den Drehwinkel zwischen zwei über ein Kopplungselement verbundene Positionierungselemente 70 berechnen und steuern.

Durch die relative Winkelstellung - gegebenenfalls in Verbindung mit der jeweiligen Längeneinstellung - der Positionierungselemente 70 ist eine freie Positionierung der Emissionsquelle 20 und des Detektors 30 auf alle Drehpunkte innerhalb der Untersuchungsregion gegeben. Beispielhaft sind drei mögliche Drehpunkte 1,2,3 in der FIG 7 eingezeichnet, die durch eine geeignete Wahl der Winkelstellungen der Positionierungselemente 70 relativ zueinander frei wählbar sind. Kombinationen aus verschieb- und drehbaren Positionierungselementengegebenenfalls in Verbindung mit der Verwendung von Kopplungselementen- gewährleisten eine freie Positionierung der Drehpunkte in einer dreidimensionalen Untersuchungsregion.

Das medizinische Bildaufnahmesystem 10 gewährleistet durch die freie Positionierbarkeit der Strahlenverläufe 90 bis 98 (nicht eingezeichnet) bezüglich der frei wählbaren Drehpunkte eine maximal mögliche Überlappung der Strahlenverläufe 90 bis 98. Hierdurch ist die Erzeugung eines gegenüber dem Stand der Technik größeren Rekonstruktionsvolumens 40 (nicht eingezeichnet) möglich.

## Patentansprüche

1. Verfahren zur Generierung eines medizinischen Bildes (110) von einem Objekt (100), wobei mindestens eine Emissionsquelle (20) und mindestens ein Detektor (30) eines medizinischen Bildaufnahmesystems (10) relativ zueinander angeordnet sind und die Strahlung der Emissionsquelle (20) durch ein Objekt (100) abschwächbar ist und die abgeschwächte Strahlung vom Detektor (30) detektiert und die vom Detektor (30) detektierte Strahlung zur Generierung des medizinischen Bildes (110) dient, mit folgenden Schritten:
a.) Unabhängige Positionierung der Emissionsquelle (20) und des Detektors (30) bei jeder Objektbestrahlung (90) relativ zueinander und im Bezug auf das Objekt (100), wobei die relative Position und Ausrichtung der Emissionsquelle (20) und des Detektors (30) auf einen frei wählbaren Drehpunkt durch mindestens ein Positionierungselement (70) erfolgt, wobei zwischen der Emissionsquelle (20) und dem Detektor (30) zumindest ein Teil des Objektes (100) anordbar ist und zumindest ein Teil der Strahlung der Emissionsquelle (20) vom Detektor (30) detektiert wird;
b.) Ermittlung der jeweiligen Position der Emissionsquelle (20) und des Detektors (30) bei jeder Objektbestrahlung (90);
c.) Erzeugung eines dreidimensionalen Rekonstruktionsvolumens (40) in mindestens einem Überlappungsbereich von zumindest zwei Objektbestrahlungen (90, 91) unter Berücksichtigung der jeweiligen Position der Emissionsquelle (20) und des Detektors (30) für jede Objektbestrahlung (90, 91);
d.) Erzeugung eines Volumenbilddatensatzes (50) auf der Grundlage des dreidimensionalen Rekonstruktionsvolumens (40) unter Berücksichtigung der zugehörigen Positionen der Emissionsquelle (20) und des Detektors (30) bei jeder Objektbestrahlung (90, 91), wobei die Emissionsquelle (20) und der Detektor (30) bei jeder Objektbestrahlung (90 bis 99) dergestalt zueinander angeordnet werden, dass die Objektbestrahlungen (90 bis 99) mindestens zwei Überlappungsbereiche und damit mindestens zwei dreidimensionale Rekonstruktionsvolumen (40,41) des Objektes (100) bilden, wobei auf der Basis der dreidimensionalen Rekonstruktionsvolumen (40,41) jeweils Volumenbilddatensätze (50,51) erzeugt werden, wobei die so gebildeten Volumenbilddatensätze (50,51) mittels eines Bild-Stitching-Verfahrens zu einem gesamten Volumenbilddatensatz (50) kombiniert werden;
e.) Generierung des medizinischen Bildes (110) bezüglich einer vorgegebenen Schnittebene des Volumenbilddatensatzes (50) .

2. Verfahren nach Anspruch 1,
**dadurch gekennzeichnet, dass** mögliche Bildverzerrungen der im Detektor (30) detektierten Objektbestrahlung (90,91) aufgrund einer möglichen Schrägstellung der Emissionsquelle (20) relativ zum Detektor (30) bei der Bilddatenverarbeitung anhand der Position der Emissionsquelle (20) relativ zu einer Detektorfläche ermittelt werden.

3. Verfahren nach einem der Ansprüche 1 oder 2,
**dadurch gekennzeichnet, dass** die Emissionsquelle (20) eine Röntgenstrahlungsquelle und der Detektor (30) eine Röntgenstrahlungsempfangseinrichtung ist.

4. Medizinisches Bildaufnahmesystem (10) zur Generierung eines medizinischen Bildes (110) gemäß einem Verfahren nach einem der vorhergehenden Ansprüchen, mit mindestens einer Emissionsquelle (20) zur Erzeugung einer Strahlung und einem Detektor (30) zur Detektion der Strahlung, wobei die vom Detektor (30) detektierte Strahlung zur Generierung eines medizinischen Bildes (110) verwendbar ist, **dadurch gekennzeichnet, dass** die relative Position und Ausrichtung der Emissionsquelle (20) und des Detektors (30) bezogen auf einen frei wählbaren Drehpunkt durch mindestens ein Positionierungselement (70) erfolgt, wobei eine Steuerung gewährleistet, dass die Emissionsquelle (20) und der Detektor (30) dergestalt positionierbar sind, dass zumindest ein Teil der Strahlung der Emissionsquelle (20) auf den Detektor (30) trifft.

5. Medizinisches Bildaufnahmesystem (10) nach Anspruch 4, **dadurch gekennzeichnet, dass** die Emissionsquelle (20) und der Detektor (30) auf einem C-Bogen (60) angeordnet sind und der C-Bogen (60) mittels des Positionierungselementes (70) vertikal und/oder waagerecht verschiebbar ist.

6. Medizinisches Bildaufnahmesystem (10) nach Anspruch 4 oder 5,
**dadurch gekennzeichnet, dass** zumindest zwei Positionierungselemente (70) durch mindestens ein drehbar gelagertes Kopplungselement miteinander verbunden sind und hierdurch eine frei Positionierung der Emissionsquelle (20) und des Detektors (30) bezogen auf einen frei wählbaren Drehpunkt gewährleistet ist.

## Claims

1. Method for generating a medical image (110) of an object (100), wherein at least one emission source (20) and at least one detector (30) of a medical imaging system (10) are arranged relative to one another and the radiation from the emission source (20) can be attenuated by an object (100) and the attenuated radiation is detected by the detector (30) and the radiation detected by the detector (30) is used for the generation of the medical image (110), having the following steps:
a.) Independent positioning of the emission source (20) and of the detector (30) relative to one another and in respect of the object (110) for each object irradiation (90), wherein the relative position and alignment of the emission source (20) and of the detector (30) to a freely selectable centre of rotation is effected using at least one positioning element (70), wherein at least a part of the object (100) can be arranged between the emission source (20) and the detector (30) and at least a part of the radiation from the emission source (20) is detected by the detector (30);
b.) Determination of the respective position of the emission source (20) and of the detector (30) for each object irradiation (90);
c.) Generation of a three-dimensional reconstruction volume (40) in at least one overlap area of at least two object irradiations (90, 91) taking into consideration the respective position of the emission source (20) and of the detector (30) for each object irradiation (90, 91);
d.) Generation of a volume image data record (50) on the basis of the three-dimensional reconstruction volume (40) taking into consideration the associated positions of the emission source (20) and of the detector (30) for each object irradiation (90, 91), wherein the emission source (20) and the detector (30) are arranged in respect of one another for each object irradiation (90 to 99) such that the object irradiations (90 to 99) form at least two overlap areas and thus at least two three-dimensional reconstruction volumes (40, 41) of the object (100), wherein based on the three-dimensional reconstruction volumes (40, 41) volume image data records (50, 51) are generated in each case, wherein the volume image data records (50, 51) formed in this way are combined by means of an image stitching method to form a complete volume image data record (50);
e.) Generation of the medical image (110) relative to a predetermined section plane of the volume image data record (50) .

2. Method according to claim 1,
**characterised in that** possible image distortions of the object irradiation (90, 91) detected in the detector (30) are determined on the basis of a possible inclination of the emission source (20) relative to the detector (30) during the image data processing based on the position of the emission source (20) relative to a detector surface.

3. Method according to one of claims 1 or 2,
**characterised in that** the emission source (20) is an X-ray radiation source and the detector (30) is an X-ray radiation receiving device.

4. Medical imaging system (10) for generating a medical image (110) in accordance with a method according to one of the preceding claims, having at least one emission source (20) for generating radiation and a detector (30) for detecting the radiation, wherein the radiation detected by the detector (30) can be used to generate a medical image (110), **characterised in that** the relative position and alignment of the emission source (20) and of the detector (30) relative to a freely selectable centre of rotation is effected by at least one positioning element (70), wherein a controller guarantees that the emission source (20) and the detector (30) can be positioned such that at least a part of the radiation from the emission source (20) strikes the detector (30).

5. Medical imaging system (10) according to claim 4, **characterised in that** the emission source (20) and the detector (30) are arranged on a C-arm (60) and the C-arm (60) can be moved vertically and/or horizontally by means of the positioning element (70).

6. Medical imaging system (10) according to claim 4 or 5, **characterised in that** at least two positioning elements (70) are connected to one another by at least one rotatably mounted coupling element and as a result a free positioning of the emission source (20) and of the detector (30) relative to a freely selectable centre of rotation is guaranteed.

## Revendications

1. Procédé de production d'une image ( 110 ) médicale d'un objet ( 100 ), dans lequel au moins une source ( 20 ) d'émission et au moins un détecteur ( 30 ) d'un système ( 10 ) d'enregistrement d'image médicale sont disposés l'un par rapport à l'autre et le rayonnement de la source ( 20 ) d'émission peut être affaibli par un objet ( 100 ) et le rayonnement affaibli est détecté par le détecteur ( 30 ) et le rayonnement détecté par le détecteur ( 30 ) sert à la production de l'image ( 110 ) médicale, comprenant les stades suivants :
a. ) mise en position indépendante l'une par rapport à l'autre et par rapport à l'objet ( 100 ) de la source ( 20 ) d'émission et du détecteur ( 30 ) pour chaque exposition ( 90 ) de l'objet au rayonnement, la position relative et l'orientation de la source ( 20 ) d'émission et du détecteur ( 30 ) s'effectuant sur un point de rotation pouvant être choisi librement par au moins un élément ( 70 ) de mise en position, au moins une partie de l'objet ( 100 ) pouvant être mise entre la source ( 20 ) d'émission et le détecteur ( 30 ) et on détecte par le détecteur ( 30 ) au moins une partie du rayonnement de la source ( 20 ) d'émission ;
b. ) détermination de la position de la source ( 20 ) d'émission et du détecteur ( 30 ) pour chaque exposition ( 90 ) de l'objet à du rayonnement ;
c. ) production d'un volume ( 40 ) de reconstruction en trois dimensions dans au moins une région de chevauchement d'au moins deux expositions ( 90, 91 ) de l'objet à du rayonnement en tenant compte de la position respective de la source ( 20 ) d'émission et du détecteur ( 30 ) pour chaque exposition ( 90, 91 ) de l'objet à du rayonnement ;
d. ) production d'un ensemble ( 50 ) de données d'image en volume sur la base du volume ( 40 ) de reconstruction en trois dimensions en tenant compte des positions propres de la source ( 20 ) d'émission et du détecteur ( 30 ) pour chaque exposition ( 90, 91 ) de l'objet à du rayonnement, la source ( 20 ) d'émission et le détecteur ( 30 ) étant, pour chaque exposition ( 90 à 99 ) de l'objet à du rayonnement, disposés l'un par rapport à l'autre, de manière à ce que les expositions ( 90 à 99 ) de l'objet à du rayonnement forment au moins deux régions de chevauchement et ainsi au moins deux volumes ( 40, 41 ) de reconstruction en trois dimensions de l'objet ( 100 ) dans lequel, sur la base des volumes ( 40, 41 ) de reconstruction en trois dimensions, on produit, respectivement, des ensembles ( 50, 51 ) de données d'image en volume, les ensembles ( 50, 51 ) de données d'image en volume ainsi formés étant combinés au moyen d'un procédé de stitching d'image en un ensemble ( 50 ) de données d'image en volume d'ensemble ;
e. ) production de l'image ( 110 ) médicale se rapportant à un plan de coupe donné à l'avance de l'ensemble ( 50 ) de données d'image en volume.

2. Procédé suivant la revendication 1,
**caractérisé en ce que** l'on détermine, à l'aide de la position de la source ( 20 ) d'émission par rapport à une surface du détecteur ( 30 ) lors du traitement des données d'image, d'éventuelles distorsions d'image de l'exposition ( 90, 91 ) de l'objet au rayonnement détectées dans le détecteur ( 30 ) en raison d'une position éventuellement inclinée de la source ( 20 ) d'émission par rapport au détecteur ( 30 ).

3. Procédé suivant l'une des revendications 1 ou 2,
**caractérisé en ce que** la source ( 20 ) d'émission est une source de rayonnement X et le détecteur ( 30 ) est un dispositif de réception de rayonnement X.

4. Système ( 10 ) d'enregistrement d'image médicale pour la production d'une image ( 110 ) médicale suivant un procédé suivant l'une des revendications précédentes, comprenant au moins une source ( 20 ) d'émission pour la production d'un rayonnement et un détecteur ( 30 ) pour la détection du rayonnement, le rayonnement détecté par le détecteur ( 30 ) pouvant être utilisé pour la production d'une image ( 110 ) médicale, **caractérisé en ce que** la position relative et l'orientation de la source ( 20 ) d'émission et du détecteur ( 30 ) par rapport à un point de rotation pouvant être choisi librement s'effectue par au moins un élément ( 70 ) de mise en position, dans lequel une commande assure que la source ( 20 ) d'émission et le détecteur ( 30 ) peuvent être mis en position de manière à ce qu'au moins une partie du rayonnement de la source ( 20 ) d'émission arrive sur le détecteur ( 30 ).

5. Système ( 10 ) d'enregistrement d'image médicale suivant la revendication 4,
**caractérisé en ce que** la source ( 20 ) d'émission et le détecteur ( 30 ) sont disposés sur un arceau ( 60 ) en C et l'arceau ( 60 ) en C peut être déplacé verticalement et/ou horizontalement au moyen de l'élément ( 70 ) de mise en position.

6. Système ( 10 ) d'enregistrement d'image médicale suivant la revendication 4 ou 5,
**caractérisé en ce qu'**au moins deux éléments ( 70 ) de mise en position sont reliés entre eux par au moins un élément d'accouplement monté tournant et, ainsi, une liberté de mise en position de la source ( 20 ) d'émission et du détecteur par rapport à un point de rotation pouvant être choisi librement est assurée.
